# EUROPEAN PATENT APPLICATION

(11) **EP 4 659 705 A1**
(43) Date of publication of application: **10.12.2025**
(21) Application number: 24180238.8
(22) Date of filing: 05.06.2024
(51) Int. Cl.: A61F 2/08, A61B 17/03

(54) **METHOD, SET, PREPARATION DEVICE, SURGICAL CLAMP, SYSTEM AND USE OF A PREPARATION DEVICE OR SURGICAL CLAMP**

(71) Applicant: ZuriMED Technologies AG, 8008 Zürich (CH)
(72) Inventor: BANZET, Pol, Zürich (CH); BACHMANN, Elias, Zürich (CH); LI, Xiang, Zürich (CH)
(74) Representative: Vossius & Partner Patentanwälte Rechtsanwälte mbB

(57) **Abstract**

A method of providing a composite implant (20) is provided. In the method, a preparation device (10) is provided. The preparation device includes a viscoelastic material (56, 110) configured to be penetrated by a felting needle. A primary and a secondary implant (14, 107; 13, 108) are also provided. The primary implant and the secondary implant are arranged on top of each other and on top of the viscoelastic material. Then, the primary implant is felted to the secondary implant with a felting device (15). The felting device comprises a felting needle that moves reciprocally. The felting needle penetrates the primary implant, the secondary implant and the viscoelastic material while felting. Thereby, fibers of the primary implant a pushed into the secondary implant.

## Description

The present disclosure relates to a method, a set, a preparation device, a surgical clamp, a system and a use of a preparation device or surgical clamp.

The rotator cuff is a group of muscles and their tendons that act to stabilize the human shoulder and allow for its extensive range of motion. The rotator cuff includes four muscles, i.e. the supraspinatus muscle, the infraspinatus muscle, the teres minor muscle, and the subscapularis muscle. Each of the muscles includes a corresponding tendon. The four tendons of these muscles converge to form the rotator cuff. Most tears occur in the supraspinatus tendon, but other parts of the rotator cuff may also be involved. In many cases, torn tendons begin by fraying. As the damage progresses, the tendon can completely tear, sometimes with lifting a heavy object.

Repair of such tears may include open surgery, an arthroscopic repair, or a combination of both. In both approaches, the repair may include fixating a tendon repair implant in the rotator cuff. Such implants may improve healing of a torn tendon and may improve an initial mechanical augmentation of the tendon. Further, rotator cuff repair may include implanting an autograft, xenograft, allograft or an artificial tendon.

Typically, rotator cuff repair involves an implantation of bone anchors. Sutures may attach the partially or completely torn tendon to the bone anchor. Since the sutures attach the tendon to the bone, they are load bearing. However, sutures, in particular load-bearing sutures, may cause a phenomenon called 'cheese-wiring'. Cheese-wiring is a problem that may occur whenever sutures are used, e.g. when fixating an implant and/or when sutures pass through tissue. The sutures cause local stress peaks, in particular when the implant needs to provide (initial) mechanical augmentation. If the stress becomes too high the sutures may start cutting through tissue (regardless of whether an implant is additionally attached). This problem may occur in any attachment of implants to tissue but are particularly relevant for tendons as these are regularly exposed to high mechanical loads. Furthermore, implantation of additional implants or patches into soft tissue can prove challenging or time consuming.

An example tendon repair implant is disclosed in WO 2016/205186 A1. An example implant that is currently marketed is the Regeneten manufactured by Smith and Nephew. Typically, such implants are fixated to the tendons of a patient using sutures.. While other attachment techniques, such as stapling, are known that may cause less (or no) cheese-wiring, these techniques may not provide a strong mechanical connection on their own (especially for tendons). Existing implants do not address the problem of cheese-wiring directly, and instead promote promoting healing (such as by using the Regeneten implant).

Recently, the present applicant has developed a new affixation technique ("Fiber Locker"). In this technique, a felt material is felted to tissue. The felt material comprises a multitude of fibers in the form of a matting or felted patch. Some of the fibers of the felted patch are pushed or pulled through the implant into the soft tissue producing a connection between the felt material and the soft tissue (see e.g., WO 2020/227838 A1). When using such a felting needle, the tissue surrounding the needle might need to be shielded from it, both for protecting the potentially sensitive tissue from the needle, and for protecting the potentially fragile needle from surrounding hard tissue or hard tools. Additionally, in the case of repairing or augmenting loose soft tissue, it is necessary to hold the tissue and the nonwoven surgical implant in place to guarantee proper penetration and fiber transportation by the reciprocating needle.

WO 2020/227838 A1 discloses a clamping device in the form of a forceps and comprising a first clamping member and a second clamping member. The second clamping member comprises a plurality of brush fibers to provide back pressure against loose tissue. However, these solutions make use of materials that are supposed to stay intact and that are relatively hard. They do not provide a perfectly continuous back pressure to the soft tissue and may damage the reciprocating needle.

Further, it may be desirable to connect implants to each other prior to an operation. For example, a feltable implant could be connected to an autograft, xenograft, allograft, tendon repair implant or an artificial tendon prior to an operation.

According to a first aspect of the disclosure, a method of providing a composite implant is provided. In the method, a preparation device is provided. The preparation device may include a viscoelastic material configured to be penetrated by a felting needle. A primary and a secondary implant are also provided. The primary implant and the secondary implant are arranged on top of each other and on top of the viscoelastic material. Then, the primary implant is felted to the secondary implant with a felting device. The felting device comprises a felting needle that moves reciprocally. The felting needle penetrates the primary implant, the secondary implant and the viscoelastic material while felting. Thereby, fibers of the primary implant are pushed into the secondary implant. Additionally, the fibers may also be pushed into the viscoelastic material.

Preferably the viscoelastic material has a thickness that is larger than an amplitude of the movement of the felting needle.

The viscoelastic material provides a backpressure which supports the felting while at the same time allowing the needle to penetrate. The viscoelastic material may provide the advantage that it mimics the behavior of soft tissue more closely. The viscoelastic material provides a continuous surface with a continuous back pressure. The viscoelastic behavior of the material may close any gaps caused by an earlier penetration. Thus, even during repeated motion, the back pressure remains substantially constant.

The present disclosure allows for a movement of the needle through the viscoelastic material that is smoother. Such viscoelastic materials may in addition be molded. Further, viscoelastic materials are available in a wide range of hardness. Its hardness is tuned to allow easy penetration of the reciprocating needle and the microfibers it transports, while having a weak anchoring force of the fibers to allow easy removal of the nonwoven implant from the backing material after felting.

A composite implant as mentioned herein may refer to the attachment or connection of two implants to each other. For example, a composite implant may be formed if a primary implant is attached to a secondary implant, in particular by felting.

A viscoelastic material as mentioned herein may refer to materials that exhibit both viscous and elastic characteristics when undergoing deformation. The elastic properties may be defined by a Youngs Modulus of the material which may be 0.02-50 MPa; preferably 0.1-15 MPa; most preferably 0.2-5 MPa. Example materials may include the following Youngs Modulus: agar agar: 0.03 - 0.7 MPa, silicone rubber: 1 - 50 MPa, synthetic gelatin: 0.1-1 MPa, gelatin: 0.02 - 0.35 MPa.

Viscoelastic material as mentioned herein may mean soft enough to be penetrated easily by the needle and/or conformable and elastic enough to retain its shape after being penetrated by the needle. Example felting needles have a width or diameter of less than 2 mm, less than 1 mm, less than 0.8 mm, preferably less than 0.6 mm, most preferably less than 0.4 mm. The felting needle may have a length from 5 to 30 mm and may or may not include barbs. In some embodiments the needle may have a length of at least 4, 6, 8, or 10 mm. The device may be configured to expose 1-20mm; 4-12mm; 6-8mm of the needle. The device may include a mechanism that converts a rotational motion into a linear reciprocal motion such as a scotch yoke. The linear motion may have a length of 1-20mm, 4-12mm, or 6-8mm. The needle may include a tapering tip, e.g. a conical tip. Advantageously, the viscoelastic material can be penetrated by such a needle without damaging the needle, in particular without substantial buckling. Further, the viscoelastic material provides some resistance to the motion of the needle. The viscoelastic material may retain some of the pushed fibers.

The primary or secondary implant may be or comprise a tendon repair implant, an artificial tendon, an autograft, xenograft, and/or allograft. The primary or secondary implant may be or comprise a nonwoven felt. In one embodiment, the primary implant may comprise a tendon repair implant and the secondary implant may be a nonwoven felt. The secondary implant may improve the mechanical properties while the primary implant may improve healing or vice versae.

A reciprocal motion of the felting device and the felting device are described for example in WO 2023/099409 A1.

In a preferred embodiment, the viscoelastic material comprises or is made of silicone synthetic gelatin, hydrolyzed collagen, agar agar based gelatin, collagen based natural gelatin, inorganic or oil-based gelatin, or natural or synthetic rubber. Particularly preferred embodiments are silicone and gelatin (synthetic and agar agar based). These materials provide a sufficient backpressure while at the same time allowing the needle to penetrate. This may mimic the mechanical behavior of soft tissue closely and may thus provide an optimal resistance when felting.

In a preferred embodiment, the viscoelastic material additionally comprises natural fibers. These natural fibers may be added prior to or during molding of the viscoelastic material. The natural fibers that may be added include in particular silk, cotton, collagen fibers, or synthetic fibers, in particular PET, POM, Glass, PTFE, PGA, PLA, PP, or PA fibers. The fibers may be chopped and dispersed. In some embodiments, the fibers may be arranged along a specific direction, i.e., the fibers may be arranged in parallel. The fibers may help making the material tougher, in particular less prone to tear. The chopping may help in letting the needle and microfibers of the nonwoven pass-through and change the material's properties such as making it more isotropic.

In a preferred embodiment, the method comprises the step of placing the primary implant in a receptacle of the viscoelastic material. The receptacle may be sized and shaped to receive the primary implant and/or the secondary implant. The receptacle may hold the primary implant and/or the secondary implant in place during felting, simplifying the felting step.

The receptacle may hold a liquid, such as a saline solution. Some implants, such as tendon repair implants or autograft, xenograft, allograft, or an artificial tendon may deteriorate if they are not kept in a liquid environment. In order to avoid this, the presently proposed method allows to keep those implants wet to prevent such a deterioration. Sized and/or shaped for receiving the primary and/or secondary implant may mean that the receptacle has the same outer dimensions as the primary and/or secondary implant. In particular, the receptacle may have the same length and/or width dimensions as the primary and/or secondary implant. In some embodiments, the receptacle may even have the same depth though it is preferred to have a larger depth for the receptacle.

The receptacle may hold the primary implant and/or the secondary implant in place during felting through its shape. In addition, the primary and/or secondary implant may be clamped in the receptacle, e.g., using prongs.

In a preferred embodiment, the primary implant comprises a nonwoven material and the primary implant may be arranged at least on one or more edges of the secondary implant and wherein the one or more edges of the primary implant may be felted to the secondary implant. Thereby, only an outer edge of the secondary implant is felted to the primary implant. This allows for a quicker attachment and a reliable connection. The composite implant can then be implanted as a single piece. The primary and the secondary implant may be connected over the entire surface in an alternative (instead of only at the edges of the secondary implant).

In a preferred embodiment, the viscoelastic material comprises a longitudinally extending cut-out. The longitudinally extending cut-out may be sized and shaped to receive the secondary implant. A longitudinally extending cut-out, may be particularly suitable for artificial tendons or autograft, xenograft, or allograft tendons. Preferably, the longitudinally extending cut-out has the shape of a tendon, preferably only partially.

In a preferred embodiment, the method comprises additionally the step of molding the viscoelastic material. The step may occur prior to arranging the primary and the secondary implant on top of each other and on the viscoelastic material. For example, an operator in a clinical environment may be provided with a set of forms and, depending on the planned composite implant, may choose to mold a form that is specifically suitable for a certain primary and/or secondary implant.

In a preferred embodiment, the method additionally comprises the step of cutting the receptacle and/or the longitudinally extending cut-out into the viscoelastic material. The viscoelastic material may be cut, e.g., using a sharp tool. The viscoelastic material may include precut sections indicating possible receptacles, e.g. for predefined use cases. In some embodiments, the viscoelastic materials may comprise 2, 3, 4, 5 or more receptacles. The receptacles may be arranged next to each other or may overlap. If the receptacles overlap, they may have different depths. For example, the primary implant may be smaller than the secondary implant and a first receptacle may be sized and shaped for the primary implant and the second receptacle may be sized and shaped for the secondary implant. Thereby, the primary and secondary implants may be positioned and fixated to each other according to a predetermined setting.

In a preferred embodiment, the secondary implant is a graft tendon, a compressed autograft tendon patch, a suture, or a suture tape. Though the composite implant may be felted to soft tissue as described in earlier applications of the present applicant, the present disclosure also encompasses the option of suturing the composite implant to soft tissue. In further examples the implant may be felted and sutured. Example secondary implants that are currently marketed are Smith and Nephew's Regeneten; Arthrex's CuffMend; acellular dermal grafts (porcine or human for example); and mechanical patches such as the Pitch Patch. Further example secondary implants that are currently marketed are Tapestry (Zimmer), Integrity (Anika), and BioBrace (Conmed). The secondary implant may enhance healing, tissue regeneration, improve a mechanical stability of the primary implant.

A further aspect of the present invention relates to a set comprising a primary implant, a secondary implant and a preparation device for use with a felting device. The preparation device comprises a viscoelastic material configured to be penetrated by a felting needle. The viscoelastic material may have the shape of a block, i.e. the viscoelastic material is a viscoelastic block. A block is mentioned herein may describe a compact solid piece of material.

A further aspect relates to a preparation device according to the previous aspect for use with a felting device.

The viscoelastic material may have a hardness from 25 Shore OO to 5 Shore type A, preferably 35 Shore type OO and/or is made of silicone, synthetic gelatin, hydrolyzed collagen agar agar based gelatin, collagen based natural gelatin, inorganic, oil-based gelatin, natural and synthetic rubber. The specified hardness ensures that the viscoelastic material can be penetrated by a typical felting needle. Further, this hardness mimics a soft tissue closely. In particular embodiments, the viscoelastic material may have a hardness of a human muscle and/or a human tendon.

A felting needle as mentioned herein may be any needle that is configured to push or pull individual fibers of a felt out of the felt into another material, e.g. soft tissue or another implant. The felting needle may comprise barbs or, alternatively, a flat or notched tip that can push individual fibers.

In a preferred embodiment, the viscoelastic material comprises a first receptacle for receiving a primary and/or a secondary implant.

In a preferred embodiment the viscoelastic material has a thickness of at least 10 mm, preferably at least 15 or 20 mm.

In a preferred embodiment the preparation device includes a frame for holding the viscoelastic material. This may hold the viscoelastic material in place during felting and may simplify handling and protect the viscoelastic material. The frame may include a fixation, in particular fixation prongs for fixing the viscoelastic material to the frame.

Further, the fixation may additionally be used to fixate one or both of the implants to the viscoelastic material. In other embodiments, the preparation device additionally comprises one or more holding prongs for holding the implants in place during felting.

The preparation device, in particular the viscoelastic material may include markings. The markings may indicate where sutures need to be attached to one or all of the implants. Thereby, sutures can be accurately positioned on the implants prior to in vivo attachment.

In a preferred embodiment, the viscoelastic material comprises a longitudinally extending cut-out for receiving a secondary implant.

A surface of the viscoelastic material may be textured. The texturing may increase friction between a primary implant or secondary implant and the viscoelastic material. The texturing may include dimples, notches, ridges, and/or a roughened surface. Thereby, the primary and/or secondary implants may be held in place more easily due to a higher friction between the implants and the viscoelastic material. This may be particularly useful if one or both of the implants is wetand/or must be maintained within a (saline) solution.

A further aspect of the present invention relates to a surgical clamp comprising a first jaw having a retainer. The retainer holds the preparation device according to any one of the preceding claims. The retainer may be releasably connectable to the first jaw. A surgical clamp allows holding one or more implants in place and felting the one or more implants to each other or to a soft tissue. The preparation device including the viscoelastic material provides a proper back pressure for the felting. The viscoelastic material provides a continuous back pressure and produces a risk of damaging the moving needle. The viscoelastic materials provided above allow for an easy shaping e.g. using molding, can be fully biocompatible and are available in a wide range of hardness.

In a preferred embodiment, the retainer is releasably connectable to the first jaw. This enables exchanging the preparation device, in particular the viscoelastic material between uses. In a preferred embodiment, the retainer includes an opening, through which the preparation device is accessible when fixated in the retainer. The opening may be for removing and inserting the viscoelastic material. In a preferred embodiment, the retainer includes a flap or closure for closing the opening at least partially. In a preferred embodiment, the flap or closure may hold the viscoelastic material in combination with the remaining parts of the retainer, including e.g. a recess, in place. The flap or closure and the remaining parts of the retainer may form a cavity in which the viscoelastic material is held.

In a preferred embodiment, the surgical clamp comprises a second jaw. The first and second jaw may be movable toward and away from each other. Each jaw may have a clamping surface that faces the opposing jaw. The first jaw and the second jaw may have an open and a closed position. The second jaw may comprise an opening for felting a primary implant. Such clamps can clamp a primary and/or secondary implant in between the first and second jaw and provide a suitable backpressure using the preparation device held in the first jaw.

In a preferred embodiment, the primary implant is attached to the second jaw. For example, the first and/or second jaw may comprise jaw attachment clamps for holding the implants prior to the jaws moving to the closed position.

A further aspect of the present invention relates to a use of a surgical device or surgical clamp as described above.

A further aspect relates to a system including a felting device and a preparation device or surgical clamp as described above.

Further features and advantages of the invention shall be described by means of detailed descriptions of embodiments with reference to the figures.
Figure 1 shows a preparation device.
Figures 2A to 2C show different embodiments of preparation devices including receptacles and cut-outs.
Figures 3A to 3B show a preparation device with a frame.
Figures 4A to 4D show a method of manufacturing a composite implant using a preparation device.
Figure 5 shows a felting of a primary implant to a secondary implant in a detailed view.
Figures 6A and 6B show an example method of attaching a composite implant to a shoulder joint of a human.
Figure 7 shows a retainer of a surgical clamp.
Figure 8 shows the retainer of figure 7 in a felting process.
Figures 9A and 9B show a surgical clamp with a retainer for a preparation device.

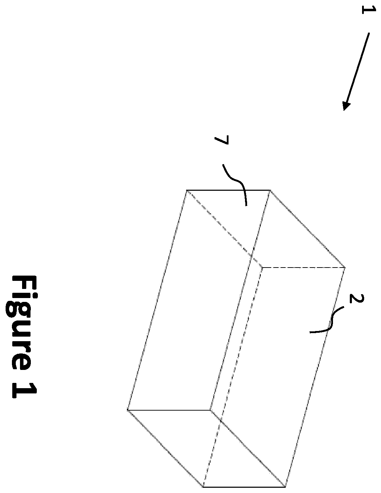

Figure 1 shows a preparation device 1. The preparation device 1 shown in figure 1 consists of a viscoelastic material 7 in the form of a rectangular block of silicone. Such a rectangular block of silicone may be obtained e.g. using molding. The block of silicone has a Youngs Modulus of 0.5 MPa and a hardness of 40 Shore A. Silicone is highly inert, chemically stable, and biocompatible and thus particularly suitable for working with medical implants. The silicone may be injection molded, extruded or 3D printed.

The block has an upper side 2 on which a user can work. The block may be used on a table, e.g. on a lab bench, and may have corresponding measurements. For example, the block may have a length of 30 cm, a width of 20 cm, and a thickness of 5 cm. The block includes chopped silk fibers to increase the toughness of the viscoelastic block.

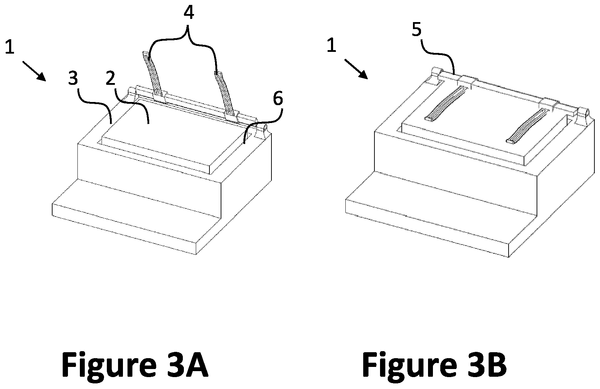

The block may be used directly on a working surface to prepare a composite implant. However, in some embodiments, the preparation device 1 may additionally include a frame 3 for holding the viscoelastic material as shown in figures 3A and 3B. The frame 3 may include a depression 6 that receives the rectangular block of silicone and has a corresponding size. In addition, the frame 3 includes holding prongs 4. The holding prongs 4 may be, as shown in figures 3A and 3B, fixated to a pivot pin 5 and extend from the pivot pin 5 radially outwardly. The pivot pin 5 may be rotationally biased towards a downward position of the prongs 4. The pivot pin 5 may include a rotational spring that pushes the prongs against the viscoelastic material. In other embodiments, the pivot pin 5 and/or the holding prongs 4 may be fixated in a downward position. In the downward position the prongs exert pressure on the top side of the viscoelastic material.

These prongs 4 may hold an implant in place and/or may hold the viscoelastic material in place and in the frame 3. In addition, the frame 3 may include markings along one or more of the sides of the viscoelastic material. For example, the markings may include positions for implants, measurements, or a ruler.

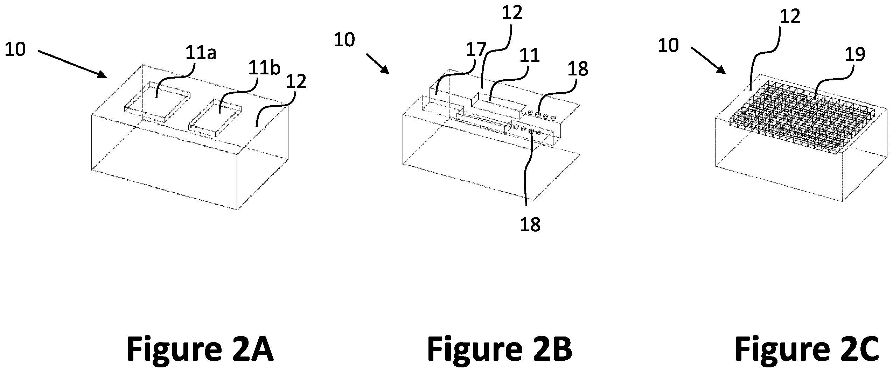

Figures 2A, 2B, and 2C show further preparation devices 10. These preparation devices 10 are similar to the preparation device 1 shown in figure 1. In addition, a top side 12 of the preparation devices 10 includes receptacles and cut-outs for preparing a composite implant. For example, figure 2A shows a preparation device 10 with two receptacles 11a and 11b. Receptacle 11a is sized and shaped to receive a primary implant, i.e. an implant including a nonwoven material, e.g. a felt. Receptacle 11b is sized and shaped to receive a secondary implant, i.e., a tendon repair implant. The receptacles 11a and 11b are recessed as compared to the top side 12 such that the primary and secondary implants can be held in a liquid, e.g., a saline solution.

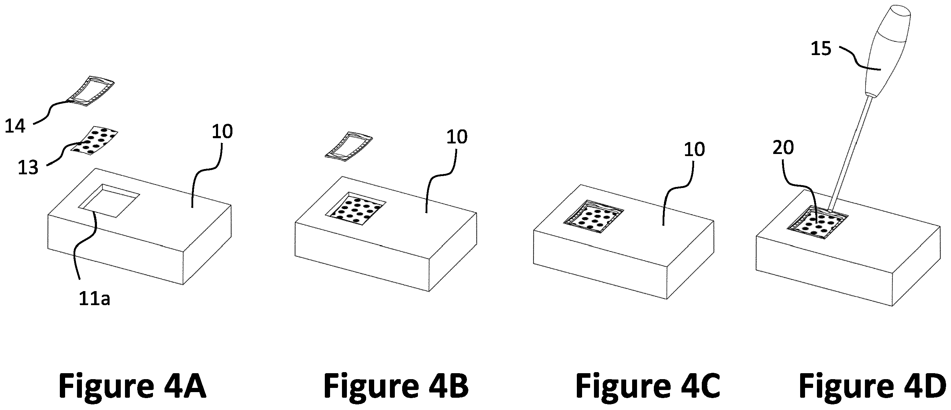

When the primary and secondary implants are held in the respective receptacles 11a, 11b then an operator may prepare the implants for a surgery outside of the body and prior to the surgery. Thus, the presently disclosed methods and devices do not need to be made during a surgery and can be used well in advance in a lab. For example, as shown in figures 4A to 4D, the operator may first place the secondary implant 13 into the receptacle 11a. The receptacle 11a has the same length and width as the secondary implant 13 as can be seen from figure 4B. A depth of the secondary implant is larger than a combined thickness of the secondary implant 13 and a primary implant 14.

The secondary implant 13 has a rectangular shape as can be seen in figures 4A to 4D. However, the secondary implant 13 is not limited to this shape and may also have any other shape depending on the application. After the secondary implant 13 is placed in the receptacle 11a, the primary implant 14 is provided. The primary implant 14 is made of nonwoven material. In the presently shown example, the primary implant 14 extends along an outer edge of the secondary implant 13. The primary implant 14 covers the outer portions of the secondary implant 13 and is laid only on the outer edges of the secondary implant 13. The primary implant 14 has the same outer dimensions but includes a window in its central portion. The primary implant 14 is laid on top of the secondary implant 13 (see figure 4C). Thereafter, as shown in figure 4D, a felting device 15 is used to felt the primary implant 14 to the secondary implant 13.

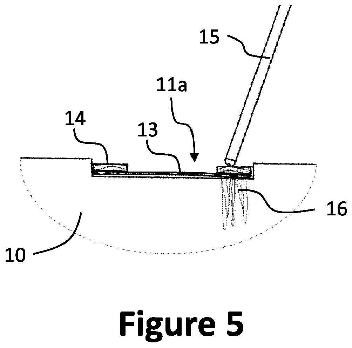

A detailed view of this felting process can be seen in figure 5. A needle (not shown) that may or may not comprise barbs is moved back and forth from a tip of the felting device 15. The needle pushes individual fibers 16 of the nonwoven material of the primary implant 14 into the secondary implant 13 and into the block of silicone below the secondary implant 13. The needle is repeatedly moved back and forth with an amplitude from 6 to 12 mm and with a frequency of 20 to 120 Hz. Thus, fibers are pushed from the nonwoven material into the secondary implant 13. Example nonwoven materials and alternative amplitudes and frequencies are disclosed in WO 2022/100832 A1. Individual nonwoven fibers 16 of the nonwoven material are retained in the secondary implant 13 and in the silicone of the preparation device 10 as can be seen in figure 5. The block of silicone provides a back pressure to the movement of the needle. Thus, the primary implant 14 and the secondary implant 13 can be held in place during felting without damaging the quickly moving needle. Other alternatives, such as air (no backpressure at all) or harder or softer materials result in either the implants undesirably moving with the needle or in damage to the needle.

In addition, individual nonwoven fibers 16 of the nonwoven material are not strongly retained within the silicone. After felting, the primary implant 14 and secondary implant 13 may be removed together without requiring a high amount of force. In particular, a user can pick up the primary and secondary implants that are felted together, i.e. a composite implant 20, for example with a surgical tool such as a clamp. A Youngs Modulus and hardness of the preparation device 10 may be tuned using chopped silk fibers as mentioned above. The chopped fibers may have a length of 10 mm or less to prevent entanglement and thus attachment of the felted nonwoven fibers 16 with the chopped fibers of the viscoelastic material.

Figure 2B shows recesses and cut-outs for primary and secondary implants that are differently shaped. For example, figure 2B illustrates a viscoelastic material that includes a longitudinally extending cut-out 17 and a receptacle 11. The longitudinally extending cut-out 17 may extend through the entire block of viscoelastic material or only through a part thereof. In this embodiment, a longitudinally extending soft tissue, such as a tendon, muscle or ligament or an artificial version thereof, can be arranged in the longitudinally extending cut-out 17. The receptacle 11 may then predefine a position of an implant that is to be felted to the longitudinally extending tissue or implant. In addition, the viscoelastic material includes markings 18 for sutures that may or may not be additionally attached to the longitudinally extending soft tissue.

Figure 2C shows a further alternative in which the viscoelastic material includes a precut section on its top side. In the shown example, a grid pattern 19 is cut into the surface of the viscoelastic material. This grid pattern 19 allows a flexible adaption of the viscoelastic material to a variety of use cases. A user can decide on the size of a receptacle, e.g., based on measurements of a primary and/or secondary implant, and remove portions of the grid accordingly such that a suitably sized receptacle is obtained.

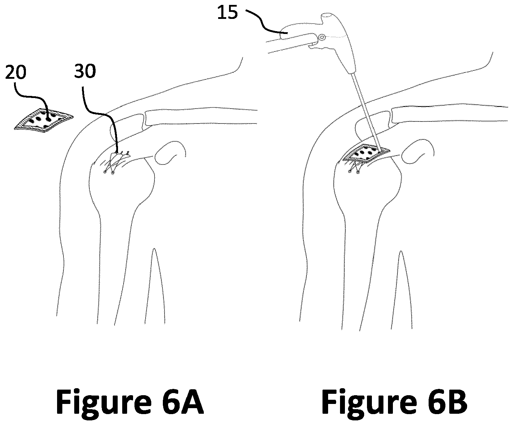

Figures 6A and 6B show a step of a shoulder repair. After manufacturing, the composite implant 20 may additionally be felted to a shoulder of a patient. In the shown example, a supraspinatus tendon is torn and was partially repaired using sutures 30 (see figure 6A). In order to further reinforce the tendon mechanically and improve healing, the composite implant 20 prepared according to the present disclosure is attached to the sutured tendon. The composite implant 20 may be attached with a felting device 15 similarly to the felting between the primary implant 14 and the secondary implant 13 described above. A tip of the felting device 15 is moved over the primary implant 14 while actuating the needle. Now, the composite implant 20 is attached to the soft tissue beneath composite implant 20, i.e. to the supraspinatus tendon. The primary implant 14 securely fixates the secondary implant 13 and provides an additional initial mechanical stability for the tendon.

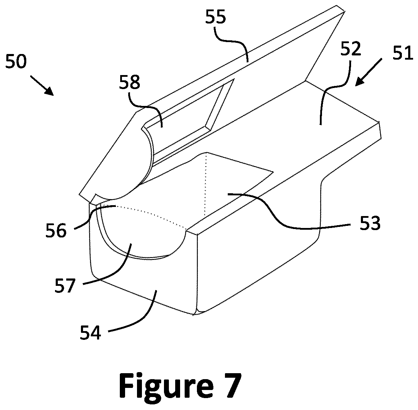

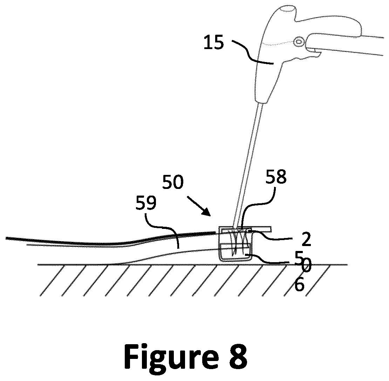

Figures 7 and 8 disclose a further embodiment of a preparation device according to the present disclosure. Figures 7 and 8 show a first clamping device 50. The clamping device 50 includes a jaw 51 having a housing 52. The housing includes two parts: a base 54 and a closure 55 (also referred to as flap). The base 54 and closure 55 form a chamber 53. A viscoelastic material 56 (indicated by the dotted lines) is held in the chamber 53. The closure 55 is movable relative to the base 54. In some embodiments, the closure 55 may be removed from the base 54 and clipped onto the base 54. However, preferably as shown in figure 7, the closure 55 is pivotably attached to the base 54. The closure 55 can be fixated in the closed position. The base 54 and closure 55 may collectively be referred to as a retainer.

In one example, the closure 55 may be closed using sutures that can be pulled to push the closure 55 and the base 54 together. In other embodiments, mechanical elements, such as latches may be used. As can be seen from figure 7, the chamber 53 includes a first opening 57. The first opening 57 may have the shape of a crescent though any other shape such as rectangular, circular, semicircular may also be suitable. The shape of the first opening 57 may correspond to a shape of a human or animal soft tissue. For example, the shape of the opening may correspond to a cross-section of a ligament or a tendon 59 (see figure 8). When the closure 55 closes the chamber 53, the closure 55 fixates a position of the tendon 59 on the viscoelastic material 56.

In some embodiments the housing 52, in particular base 54 may include a further opening opposing the first opening 57. The first opening and further openings may form a straight channel.

The closure 55 includes a second opening 58. The second opening 58 faces a top surface of the viscoelastic material 56 while the first opening 57 faces a side surface of the viscoelastic material. The first opening 57 and second opening 58 are angled with respect to each other such that the first opening allows for insertion of the implant and/or soft tissue and such that the second opening allows an operator to access the implant and/or soft tissue with a felting device 15 as shown in figure 8.

In one example, a user may first place an artificial or natural ligament or tendon 59 (or an end thereof) in the first opening 57 as shown in figure 8. The ligament or tendon 59 is placed on top of the viscoelastic material 56. Then, an implant comprising a nonwoven (e.g. primary implant 14 or composite implant 20) is placed on top of the ligament or tendon 59. The closure 55 is closed. Then, implant 20, ligament or tendon 59 and viscoelastic material 56 can be felted as e.g. shown in figure 5. The felting results in a strong, reliable connection that distributes forces acting on the implant 20, ligament and tendon 59 evenly. Thus, such a strong connection cannot just be achieved by felting in vivo but also outside of the human body and without requiring human soft tissue or hard tissue for providing a backpressure.

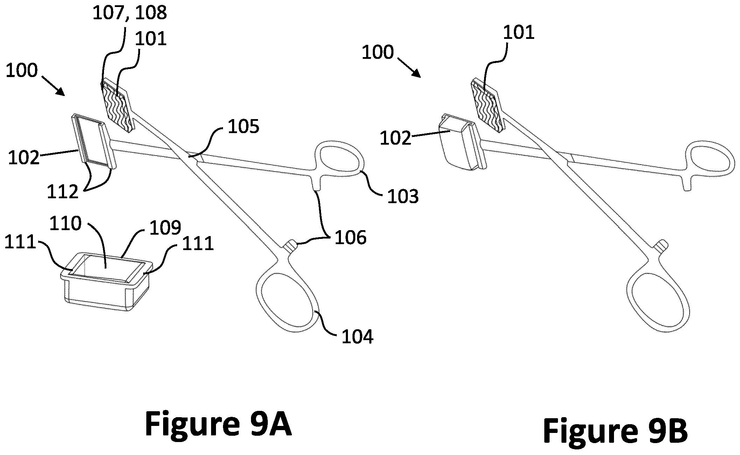

Figures 9A and 9B disclose a further embodiment of a preparation device according to the present disclosure. Similarly to the previous embodiments the preparation device includes a viscoelastic material 110 that can be used for felting implants. Figures 9A and 9B show a surgical clamp 100 with a first jaw 102 and a second jaw 101. The clamp 100 has generally the shape of a pair of scissors with the blades being replaced by the second jaw 101 and the first jaw 102. The scissors are pivotably connected at a middle section 105 of the scissors. The clamp 100 includes two handles 103 and 104 each having a ringshaped opening for a finger. The handles include a latching mechanism 106 that can fixate the clamp 100 in a closed position.

The first jaw 102 and the second jaw 101 may clamp a primary implant 107 and/or a secondary implant 108 in between them. The first jaw 102 includes C-shaped guide rails 112. C-shaped guide rails 112 are arranged opposing to each other. A retainer 109 is box shaped and open towards a top side. The retainer 109 holds the viscoelastic material 110. The retainer 109 includes guides 111 on opposing sides. The guides 111 may be slid into the C-shaped guide rails 112, as can be seen from the comparison between figures 9A and 9B. When held by the first jaw 102, the open top side of the retainer 109 is directed towards the second jaw 101.

The first jaw 102 exposes a top side of the viscoelastic material. When the first jaw 102 and the second jaw 101 clamp a primary implant 107 and a secondary implant 108 in between them, the implants are also clamped on top of the viscoelastic material. Then a user may felt the implants to each other as previously shown and the viscoelastic material provides a suitable back pressure.

Further aspects of the present disclosure are described in the following:
1. Method of providing a composite implant comprising the following steps:
   a. Providing a preparation device, wherein the preparation device includes a viscoelastic material configured to be penetrated by a felting needle;
   b. Providing a primary implant and a secondary implant;
   c. Arranging the primary implant and the secondary implant on top of each other and on the viscoelastic material; and
   d. Felting the primary implant to the secondary implant with a felting device, the felting device comprising a needle that moves reciprocally, wherein the needle of the felting device penetrates the primary implant, the secondary implant and the viscoelastic material while felting, thereby pushing fibers of the primary implant into the secondary implant.
2. Method according to aspect 1, wherein the viscoelastic material comprises or is made of silicone, synthetic gelatin, hydrolyzed collagen, agar agar based gelatin, collagen based natural gelatin, inorganic, oil-based gelatin, natural and synthetic rubber.
3. Method according to aspect 2, wherein the viscoelastic material additionally comprises natural fibers, in particular silk, cotton, collagen fibers, or synthetic fibers, in particular PET, POM, Glass, PTFE, PGA, PLA, PP, or PA fibers.
4. Method according to aspect 3, wherein the fibers are chopped and dispersed in the viscoelastic material or wherein the fibers are arranged along the same directions.
5. Method according to any one of the preceding aspects, comprising the step of placing the primary implant in a receptacle of the viscoelastic material, wherein the receptacle is sized and shaped for receiving the primary implant and/or the secondary implant and wherein the receptacle holds the primary implant and/or the secondary implant in place during felting.
6. Method according to any one of the preceding aspects, wherein the primary implant comprises a nonwoven material and wherein the primary implant is arranged on one or more edges of the secondary implant and wherein the one or more edges of the primary implant are felted to the secondary implant.
7. Method according to any one of preceding aspects, comprising the step of placing the secondary implant in a longitudinally extending cut-out sized and shaped to receive the secondary implant.
8. Method according to any one of the preceding aspects, wherein the secondary implant is a graft tendon, a compressed autograft tendon patch, a suture, or a suture tape.
9. Method according to any one of the preceding aspects, additionally comprising the step of molding the viscoelastic material.
10. Set comprising a primary implant, a secondary implant and a preparation device for use with a felting device, the preparation device comprising a viscoelastic material configured to be penetrated by a felting needle.
11. Preparation device according to aspect 10 for use with a felting device.
12. Preparation device according to aspect 10 or 11, wherein the viscoelastic material has a hardness from 25 Shore OO to 5 Shore type A, prefera bly 35 Shore type OO and/or is made of silicone, synthetic gelatin, hydrolyzed collagen, agar agar based gelatin, collagen based natural gelatin, inorganic, oil-based gelatin, natural and synthetic rubber.
13. Preparation device according to any one of aspects 10 to 12, wherein the viscoelastic material comprises a first receptacle for receiving a primary implant and/or a secondary implant.
14. Preparation device according to any one of the aspects 10 to 13, wherein the viscoelastic material has a thickness of at least 10 mm, preferably at least 15 or 20 mm.
15. Preparation device according to any one of the aspects 10 to 14, wherein the preparation device includes a frame for holding the viscoelastic material.
16. Preparation device according to any one of the aspects 10 to 15, wherein the frame includes a fixation, in particular fixation prongs, for fixing the viscoelastic material to the frame.
17. Preparation device according to any one of the aspects 10 to 16, wherein the preparation device includes markings, preferably markings for sutures.
18. Preparation device according to any one of the aspects 10 to 17, wherein the viscoelastic material comprises a longitudinally extending cut-out for receiving a secondary implant.
19. Preparation device according to any one of the aspects 10 to 18, wherein a surface of the viscoelastic material is textured such that friction between a primary implant or secondary implant and the viscoelastic material is increased.
20. Surgical clamp comprising a first jaw, the first jaw having a retainer, the retainer holding the preparation device according to any one of the preceding aspects.
21. Surgical clamp according to aspect 20, wherein the retainer is releasably connectable to the first jaw.
22. Surgical clamp according to any one of aspect 20 to 21, wherein the retainer includes an opening, through which the preparation device is accessible when fixated in the retainer.
23. Surgical clamp according to any one of the aspects 20 to 22, comprising a second jaw, wherein the first jaw and second jaw are movable toward and away from each other, each jaw having a clamping surface that faces the other jaw, and the first jaw and the second jaw having an open and a closed position; and the second jaw comprising an opening for felting a primary implant.
24. Surgical clamp according to aspect 23, wherein a primary implant is attached to the second jaw.
25. Use of a preparation device or surgical clamp according to any one of the aspects 10 to 24 for attaching a primary implant to a secondary implant.
26. System including a felting device and a preparation device or surgical clamp according to any one of the aspects 10 to 24.

## Claims

1. Method of providing a composite implant (20) comprising the following steps:
a. Providing a preparation device (10), wherein the preparation device (10) includes a viscoelastic material (56, 110) configured to be penetrated by a felting needle;
b. Providing a primary implant (14, 107) and a secondary implant (13, 108);
c. Arranging the primary implant (14, 107) and the secondary implant (13, 108) on top of each other and on the viscoelastic material (56, 110); and
d. Felting the primary implant (14, 107) to the secondary implant (13, 108) with a felting device (15), the felting device (15) comprising a needle that moves reciprocally, wherein the needle of the felting device (15) penetrates the primary implant (14, 107), the secondary implant (13, 108) and the viscoelastic material (56, 110) while felting, thereby pushing fibers (16) of the primary implant (14, 107) into the secondary implant (13, 108).

2. Method according to claim 1, wherein the viscoelastic material (56, 110) comprises or is made of silicone, synthetic gelatin, hydrolyzed collagen, agar agar based gelatin, collagen based natural gelatin, inorganic, oil-based gelatin, natural and synthetic rubber.

3. Method according to claim 2, wherein the viscoelastic material (56, 110) additionally comprises natural fibers (16), in particular silk, cotton, collagen fibers (16), or synthetic fibers (16), in particular PET, POM, Glass, PTFE, PGA, PLA, PP, or PA fibers (16).

4. Method according to claim 3, wherein the fibers (16) are chopped and dispersed in the viscoelastic material (56, 110) or wherein the fibers (16) are arranged along the same directions

5. Method according to any one of the preceding claims, comprising the step of placing the primary implant (14, 107) in a receptacle (11a, 11b) of the viscoelastic material (56, 110), wherein the receptacle (11a, 11b) is sized and shaped for receiving the primary implant (14, 107) and/or the secondary implant (13, 108) and wherein the receptacle (11a, 11b) holds the primary implant (14, 107) and/or the secondary implant (13, 108) in place during felting.

6. Method according to any one of the preceding claims, wherein the secondary implant (13, 108) is a graft tendon, a compressed autograft tendon patch, a suture, or a suture tape.

7. Set comprising a primary implant (14, 107), a secondary implant (13, 108) and a preparation device (10) for use with a felting device (15), the preparation device (10) comprising a viscoelastic material (56, 110) configured to be penetrated by a felting needle and the primary implant (14, 107) comprising a nonwoven material the set optionally comprising a felting device (15).

8. Preparation device according to claim 7 for use with a felting device (15).

9. Preparation device according to claim 7 or 8, wherein the viscoelastic material (56, 110) has a hardness from 25 Shore OO to 5 Shore type A, preferably 35 Shore type OO.

10. Preparation device according to any one of claims 7 to 9, wherein the viscoelastic material (56, 110) comprises a first receptacle for receiving a primary implant (14, 107) and/or a secondary implant (13, 108).

11. Surgical clamp comprising a first jaw (102), the first jaw (102) having a retainer (109), the retainer (109) holding the preparation device (10) according to any one of claims 7 to 10.

12. Surgical clamp according to claim 11, wherein the retainer (109) includes an opening (57, 58), through which the preparation device (10) is accessible during felting when the preparation device (10) is fixated in the retainer (109).

13. Surgical clamp according to claim 12, comprising a second jaw (101), wherein the first jaw (102) and second jaw (101) are movable toward and away from each other, each jaw having a clamping surface that faces the other jaw, and the first jaw (102) and the second jaw (101) having an open and a closed position; and the second jaw (101) comprising an opening (57, 58) for felting a primary implant (14, 107).

14. Surgical clamp according to claim 13, wherein a primary implant (14, 107) is attached to the second jaw (101).
